(19) Europäisches Patentamt
European Patent Office
Office européen des brevets

(11) Veröffentlichungsnummer: **0 007 347**
**B1**

(12) # EUROPÄISCHE PATENTSCHRIFT

(45) Veröffentlichungstag der Patentschrift: **30.12.81**

(21) Anmeldenummer: **78900097.3**

(22) Anmeldetag: **28.08.78**

(86) Internationale Anmeldenummer: **PCT/CH 78/00015**

(87) Internationale Veröffentlichungsnummer: **WO 79/00116 (08.03.79** Gazette **79/5)**

(51) Int. Cl.³: **C 07 C 43/23,** C 07 C 69/90, C 07 D 213/80 // A61K31/085, A61K31/445, A61K31/60

(54) **Lipidsenkende Alkylenglykolderivate und Verfahren zu ihrer Herstellung.**

(30) Priorität: **29.08.77 CH 10498/77**

(43) Veröffentlichungstag der Anmeldung: **06.02.80 Patentblatt 80/3**

(45) Bekanntmachung des Hinweises auf die Patenterteilung: **30.12.81 Patentblatt 81/52**

(84) Benannte Vertragsstaaten: **FR**

(56) Entgegenhaltungen:
**DE-A-2 502 154**

**Chemical Abstracts, 6th Collective Index, Vol. 51-55 (1957-1961), published in 1964 (Columbus, Ohio, USA) see page 1702F**

(73) Patentinhaber: **SIEGFRIED AKTIENGESELLSCHAFT, CH-4800 Zofingen (CH)**

(72) Erfinder: **THIELE, Kurt, Rebbergstrasse 47d, CH-4800 Zofingen (CH)**
Erfinder: **AHMED, Quazi, Rigweg 5, CH-4800 Zofingen (CH)**
Erfinder: **JAHN, Ulrich, Kirchmoosstrasse 13, CH-4800 Zofingen (CH)**
Erfinder: **ADRIAN, Rudolf, Rümlisberg 470, CH-4803 Vordemwald (CH)**

(74) Vertreter: **Ritscher, Thomas, Dr. et al, RITSCHER & SEIFERT Auf der Mauer 4, CH-8001 Zürich (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Lipidsenkende Alkylenglykolderivate und Verfahren zu ihrer Herstellung

## Beschreibung

### Technisches Gebiet der Erfindung

Die Erfindung betrifft neue Alkylenglykolderivate mit biologischer Aktivität und Verfahren zur Herstellung solcher Verbindungen sowie Alkylenglykolderivate zur Verwendung als lipidsenkende Wirkstoffe.

### Stand der Technik

Chemische Mittel zur Verminderung überhöhter Cholesterin- und Triglyceridwerte im Blut sind bekannt. Eine wichtige Gruppe solcher Mittel enthält als aktive Komponente Verbindungen der Formel (10)

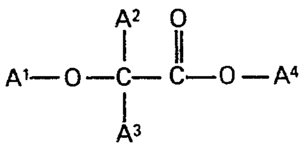

(10)

in der $A^1$ einen gegebenenfalls substituierten Arylrest, $A^2$ und $A^3$ Wasserstoff oder Alkyl und $A^4$ Wasserstoff oder organische Reste bedeuten. Verbindungen der Formel (10), in welchen $A^1$ einen gegebenenfalls substituierten Phenyl- oder Naphthylrest bedeutet, sind unter anderen in der US-PS 3 262 850 als cholesterinsenkende Mittel beschrieben und unter diesen hat insbesondere die als Clofibrinsäure ($A^1$ = p-Chlorphenyl, $A^2$ = $A^3$ = Methyl, $A^4$ = H) bezeichnete Verbindung bzw. deren Äthylester ($A^4 = C_2H_5$), das sogenannte Clofibrat, eine erhebliche klinische Bedeutung erlangt.

Zur Gruppe der durch die Formel (10) umschriebenen, als cholesterinsenkend bekannten Stoffe gehören ferner die in der DE-OS 2 356 655 beschriebenen Verbindungen ($A^1$ = p-Chlorbenzylphenyl, p-Chlorbenzyloxyphenyl und dergleichen, $A^2$ = $A^3$ = Methyl, $A^4$ = H, Alkyl oder N,N-Dialkylaminoalkylen) und die in DE-OS 2 461 069 von der Anmelderin beschriebenen Verbindungen mit bestimmten Variationen bezüglich der Gruppen $A^1$ bis $A^4$ und gegebenenfalls der Verwendung einer Schwefelbrücke anstelle der Sauerstoffbrücke zur Gruppe $A^1$.

Charakteristisch für die Struktur der bekannten cholesterinsenkenden Verbindungen der Formel (10) ist jedenfalls die 2-Oxycarbonsäure- bzw. die 2-Thiocarbonsäurestruktur und es war daher anzunehmen, dass diese Carbonsäurestruktur für die Lipidsenkungsaktivität der bekannten Verbindungen wesentlich sei.

Aufgabe der Erfindung sind neue Lipidsenker-Verbindungen, Verfahren zur Herstellung dieser Verbindungen und pharmakologische Zubereitungen, welche die neuen Lipidsenker-Verbindungen enthalten.

### Erfindung

Überraschenderweise wurde gefunden, dass Alkylenglykolderivate der Formel (1)

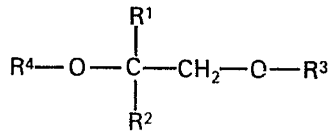

(1)

eine im Vergleich zum Clofibrat verbesserte cholesterinsenkende Wirkung selbst dann haben, wenn $R^3$ in Formel (1) Wasserstoff ist und weder $R^1$ noch $R^2$ eine zur Sauerstoffbrücke in $\alpha$-Stellung befindliche Carbonylgruppe besitzt, d.h. die 2-Oxycarbonylstruktur nicht vorhanden ist. Andererseits ist das Vorhandensein einer freien alkoholischen Hydroxylgruppe (d.h. $R^3$ = H) für die Cholesterin- bzw. Lipidsenkungswirkung offenbar insofern nicht kritisch, als auch Ester von Alkoholen der Formel (1) mit organischen Säuren Lipidsenkungswirkungen zeigen.

Gegenstand der Erfindung sind daher Verbindungen der oben angegebenen Formel (1), in der die Zeichen $R^1$ bis $R^4$ folgende Bedeutung haben:

$R^1$ ist Wasserstoff, $C_1$-$C_4$ Alkyl mit gerader oder verzweigter Kette, Phenyl oder Benzyl.

$R^2$ ist Wasserstoff oder $C_1$-$C_4$ Alkyl mit gerader oder verzweigter Kette.

Bei einer bevorzugten Gruppe von Verbindungen der Formel (1) ist $R^1$ ungleich $R^2$, insbesondere wenn beide $R^1$, $R^2$ unterschiedliche Niederalkylgruppen, z.B. Methyl/Aethyl oder Methyl/Propyl, sind.

$R^3$ ist wie erwähnt das Wasserstoffatom oder der zur Bildung eines Esters erforderliche Rest einer Carbonsäure, d.h. die Gruppe

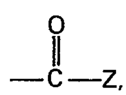

in der Z ein Alkylrest mit 1 bis 10 C-Atomen, der o-Acetyloxyphenylrest oder der 3-Pyridinylrest ist.

$R^4$ bedeutet einen Rest der Formel

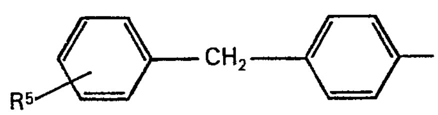

in der $R^5$ Wasserstoff, Chlor oder Methyl bedeutet.

Die Verbindung der Formel (1), in der $R^1$, $R^2$ und $R^3$ Wasserstoffatome sind und $R^4$ die p-Benzylphenylgruppe darstellt, ist als Weichmacher für bestimmte Plaste vorbeschrieben (siehe W. Thümmler, K. Thinius: Weitere Untersuchungen über Weichmacher für Zellulosetriazetat; «Plaste und Kautschuk», Band 7 (1960), S. 294–297; vgl. Chemical Abstracts, sixth collective index (1957–1961), S. 1702F; Chemical Abstracts, Band 54 (1960), Sp. 25789h).

Für die erfindungsgemässen neuen Verbindungen der Formel (1) gilt daher die Massgabe, dass $R^3$ nicht das Wasserstoffatom ist, wenn $R^4$ die

p-Benzylphenylgruppe ist und beide $R^1$, $R^2$ Wasserstoffatome sind.

Für die erfindungsgemässe Verwendung als lipidsenkender Wirkstoff gilt diese Massgabe hingegen nicht.

Gemäss einer bevorzugten Ausführungsform stellt $R^5$ einen der genannten Substituenten, insbesondere Chlor, in p-Stellung zu der Methylenbrücke zwischen den beiden Benzolkernen dar.

Die Verbindungen (1) können erfindungsgemäss aus den entsprechenden Säuren bzw. Estern der Formel (2)

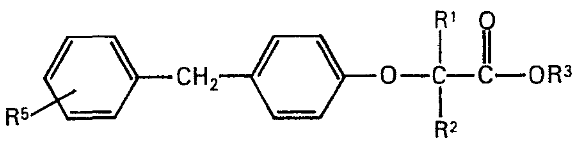

in der $R^1$ bis $R^5$ die oben angegebene Bedeutung haben, durch Reduktion, vorzugsweise mit Lithiumaluminiumhydrid in einem bezüglich der Reaktion inerten organischen Lösungsmittel, erhalten werden, gegebenenfalls mit folgender Veresterung des so erhaltenen Alkohols mit einer Carbonsäure der Formel

Z-C(O)OH

in der Z die oben angegebene Bedeutung hat, oder einem Säurechlorid einer solchen Säure. Die Säuren der Formel (2) sind entweder bekannt und beispielsweise nach den in den oben genannten Publikationen angegebenen Methoden oder in Analogie zu diesen bekannten Verbindungen herstellbar.

Die Verbindungen (1) sind erfindungsgemäss auch durch Umsetzung eines Phenols der Formel

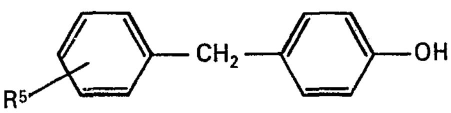

in der $R^5$ die angegebene Bedeutung hat, mit einer Verbindung der Formel

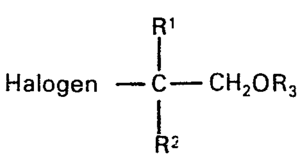 (Halogen = Cl, Br, J)

zugänglich, in der $R^1$, $R^2$ und $R^3$ die oben angegebene Bedeutung haben.

Aktivitätswerte von Verbindungen (1) und pharmakologische Zubereitungen mit diesen sind weiter unten erläutert.

Die folgenden Beispiele erläutern die Herstellung der Verbindungen (1). Prozente sind Gewichtsprozente. Die Elementaranalysewerte der Verbindungen sind in Tabelle I weiter unten zusammengestellt.

Beispiel 1

2-[4-(4′-Chlorbenzyl)-phenoxy]-äthanol der Formel

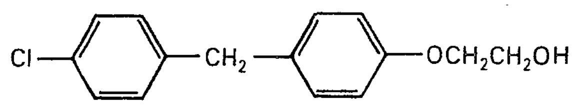

Eine Lösung von 15,2 g (0,05 Mol) 2-[4-(4′-Chlorbenzyl)-phenoxy]-essigsäureäthylester in 30 ml trockenem Diäthyläther wurde langsam mit einer Mischung aus 1,9 g (0,05 Mol) Lithiumaluminiumhydrid und 70 ml trockenem Diäthyläther versetzt. Die Mischung wurde 90 min unter Rühren zum Rückfluss erwärmt, dann abgekühlt und mit 10%iger wässriger Natriumhydroxidlösung versetzt. Das ausgefällte Salz wurde durch Filtrieren entfernt und die abgetrennte Aetherschicht nach Trocknen über Magnesiumsulfat eingedampft. Dies ergab 12,9 g rohes Zielprodukt, das aus Dichlormethan/n-Hexan umkristallisiert wurde und 10,59 g reines Zielprodukt in Form von weissen Kristallen, F 64–66 °C, ergab.

Beispiel 2

2-Methyl-2-[4-(4′-chlorbenzyl)-phenoxy]-butanol der Formel

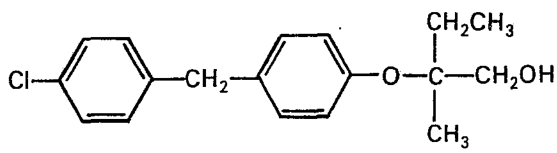

Zu einer gerührten Aufschlämmung aus 1,9 g (0,05 Mol) Lithiumaluminiumhydrid in 50 ml trockenem Diäthyläther wurde langsam eine Lösung aus 17,39 g (0,05 Mol) 2-Methyl-2-[4-(4′-chlorbenzyl)-phenoxy]-buttersäureäthylester in 40 ml trockenem Diäthyläther gegeben. Die Mischung wurde 90 min gerührt und dann mit einer 10 %igen wässrigen Natriumhydroxidlösung zersetzt. Die organische Schicht wurde abgetrennt, über Magnesiumsulfat getrocknet und unter vermindertem Druck konzentriert. Dies ergab 15 g ölartiges Rohprodukt, das beim Destillieren 13 g Zielprodukt als eine einzige flüssige Fraktion, Kp 193–196 °C/0,001 mm Hg, lieferte.

Beispiel 3

2-Phenyl-2-[4-(4′-Chlorbenzyl)-phenoxy]-äthanol der Formel

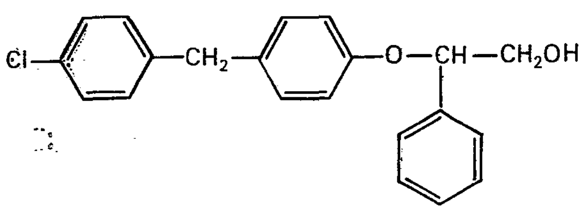

Eine Lösung aus 53,3 g (0,14 Mol) 2-Phenyl-2-[4-(4′-chlorbenzyl)-phenoxy]-essigsäureäthylester und 5,3 g (0,14 Mol) Lithiumaluminiumhydrid in

340 ml trockenem Diäthyläther wurde 90 min unter Rühren zum Rückfluss erwärmt. Durch Aufarbeiten der Reaktionsmischung wie in Beispiel 1 wurden 50 g rohes Produkt erhalten, das nach Kristallisieren aus Benzol 45 g reines Zielprodukt in Form von weissen Kristallen, F 88–89 °C, ergab.

Beispiel 4
Nicotinsäureester von 2-[4-(4'-Chlorbenzyl)-phenoxy]-äthanol der Formel

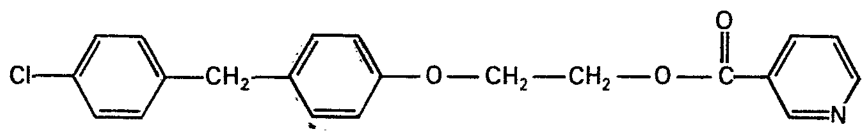

Zu einer Mischung aus 8,9 g (0,05 Mol) Nicotinoylchlorid-hydrochlorid und 10 ml trockenem Pyridin wurde langsam eine Lösung von 13,2 g (0,05 Mol) 2-[4-(4'-Chlorbenzyl)-phenoxy]-äthanol (Produkt von Beispiel 1) in 20 ml trockenem Pyridin gegeben. Die Mischung wurde 24 Std. bei Raumtemperatur (20–25 °C) gerührt. Dann wurde das Pyridin unter vermindertem Druck langsam abgedampft und der trockene Rückstand in gesättigter wässriger Natriumcarbonatlösung aufgenommen und die erhaltene Mischung mit Trichlormethan extrahiert. Die organische Phase wurde mit Wasser gewaschen, über Magnesiumsulfat getrocknet und zur Trockenheit eingedampft. Der Rückstand wurde aus Trichlormethan/n-Hexan umkristallisiert und ergab 13 g Zielprodukt in Form von weissen Kristallen, F 75–78 °C.

Beispiel 5
Hydrochlorid des Nicotinsäureesters von 2-Methyl-2-[4-(4'-chlorbenzyl)-phenoxy]-butanol der Formel

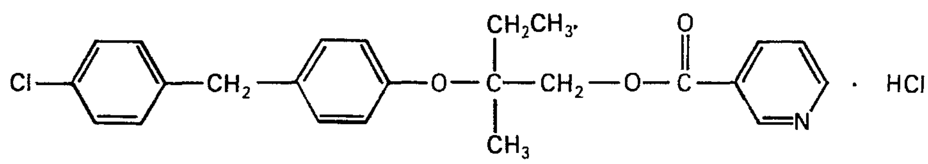

Nach der Arbeitsweise von Beispiel 4 wurden 12,6 g 2-Methyl-2-[4-(4'-chlorbenzyl)-phenoxy]-butanol (Produkt von Beispiel 2) mit einer äquimolaren Menge Nicotinoylchlorid-hydrochlorid in trockenem Pyridin verestert. Als Rückstand nach dem Abdampfen des Pyridins wurde die basische Form des Zielesters als öliges Produkt erhalten, das durch Behandlung mit HCl/Diäthyläther das entsprechende Hydrochlorid ergab und nach dem Umkristallisieren aus Methanol 10,5 g reines Zielprodukt in Form von farblosen Nadeln, F 107 °C, lieferte.

Beispiel 6
Acetylsalicylsäureester von 2-[4-(4'-Chlorbenzyl)-phenoxy]-äthanol der Formel

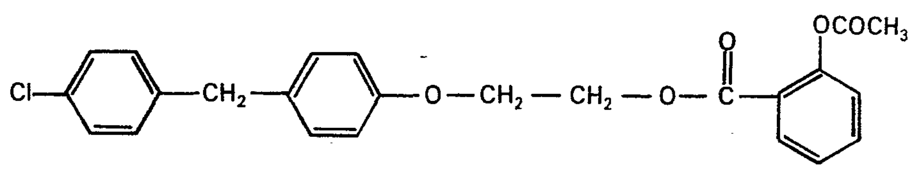

Zu einer Mischung aus 9,8 g (0,05 Mol) Acetylsalicylsäurechlorid und 12 ml Triäthylamin in 50 ml 1,2-Dichloräthan wurde eine Lösung von 13,2 g (0,05 Mol) 2-[4-(4'-Chlorbenzyl)-phenoxy]-äthanol (Produkt von Beispiel 1) in 20 ml 1,2-Dichloräthan gegeben. Die Reaktionsmischung wurde 20 Std. bei Raumtemperatur gerührt und dann filtriert. Das Filtrat wurde mit Wasser gewaschen, über Magnesiumsulfat getrocknet und unter vermindertem Druck vom Lösungsmittel befreit. Der Rückstand wurde aus Dichlormethan/n-Hexan kristallisiert und ergab 10,5 g Zielprodukt in Form von weissen Kristallen, F 67–69 °C.

Beispiel 7
Acetylsalicylsäureester von 2-Methyl-2-[4-(4'-chlorbenzyl)-phenoxy]-butanol der Formel

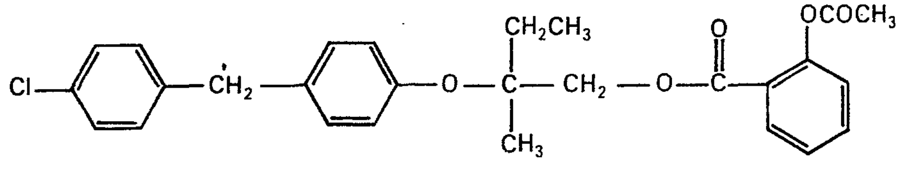

Nach der Arbeitsweise von Beispiel 6 wurde eine Mischung aus 7,9 g (0,04 Mol) Acetylsalicylsäurechlorid mit 10 ml Triäthylamin und 50 ml 1,2-Dichloräthan unter Rühren mit einer Lösung von 12,6 g (0,04 Mol) 2-Methyl-2-[4-(4'-chlorbenzyl)-phenoxy]-butanol (Produkt von Beispiel 2) in 20 ml Dichloräthan umgesetzt und aufgearbeitet. Der ölige Rückstand wurde destilliert und ergab

7,5 g Zielprodukt als fahlgelbes Oel, Kp 265–270 °C/0,05 mm Hg.

Als weitere Beispiele erfindungsgemässer Verbindungen (1) seien noch erwähnt:

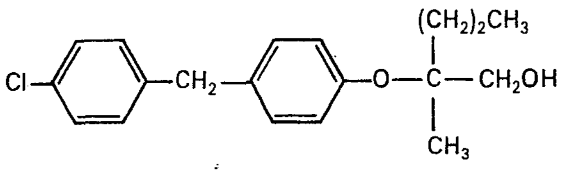

2-Methyl-2-[4-(4′-chlorbenzyl)-phenoxy]-pentanol, Kp 180–183, 5 °C/0,01 mm Hg.

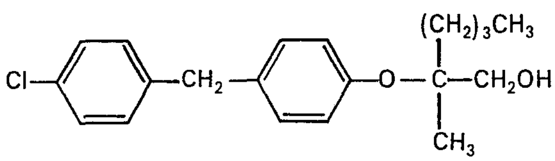

2-Methyl-2-[4-(4′-chlorbenzyl)-phenoxy]-hexanol, Kp 193–196 °C/0,001 mm Hg.

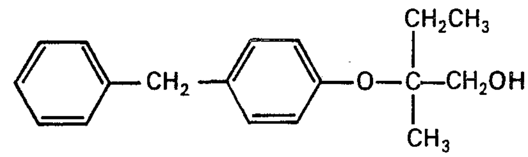

2-Methyl-2-(4-benzylphenoxy)-butanol, Kp 156–157 °C/0,001 mm Hg.

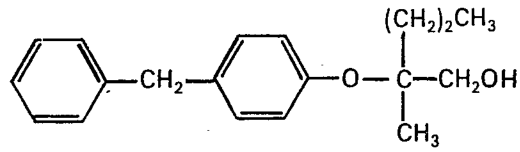

2-Methyl-2-(4-benzylphenoxy)-pentanol, Kp 165–167 °C/0,02 mm Hg.

Tabelle I

| Beisp. Nr. | Bruttoformel | C | H | O | N | Cl |
|---|---|---|---|---|---|---|
| 1 | $C_{15}H_{15}ClO_2$ | | | | | |
| | berechnet | 68.97 | 5.76 | 12.18 | | 13.49 |
| | gefunden | 69.01 | 5.79 | 11.89 | | 13.57 |
| 2 | $C_{18}H_{21}ClO_2$ | | | | | |
| | berechnet | 70.92 | 6.95 | 10.50 | | 11.63 |
| | gefunden | 71.07 | 7.02 | 10.67 | | 11.63 |
| 3 | $C_{21}H_{19}ClO_2$ | | | | | |
| | berechnet | 74.44 | 5.65 | | | 10.46 |
| | gefunden | 74.88 | 5.79 | | | 10.67 |
| 4 | $C_{21}H_{18}ClNO_3$ | | | | | |
| | berechnet | 68.52 | 5.00 | | 3.80 | |
| | gefunden | 68.68 | 5.04 | | 3.50 | |
| 5 | $C_{24}H_{24}ClNO_3 \cdot HCl$ | | | | | |
| | berechnet | 64.58 | 5.64 | | 3.14 | 15.89 |
| | gefunden | 64.18 | 5.62 | | 2.77 | 15.92 |
| 6 | $C_{24}H_{21}ClO_5$ | | | | | |
| | berechnet | 67.83 | 4.98 | 18.85 | | 8.34 |
| | gefunden | 67.92 | 4.96 | 18.77 | | 8.54 |
| 7 | $C_{27}H_{27}ClO_5$ | | | | | |
| | berechnet | 67.44 | 5.83 | | | 7.60 |
| | gefunden | 69.99 | 5.99 | | | 7.85 |

Die Toxizitätswerte und Aktivitäten der obigen Verbindungen (1) wurden im Tierversuch nach den üblichen und beispielsweise in den obigen Publikationen beschriebenen Methoden geprüft und mit den entsprechenden Werten des bekannten Cholesterinsenkers Clofibrat verglichen:

1. Toxizität

Der LD 50-Wert bei Mäusen in mg aktiver Substanz pro kg Körpergewicht, der für Clofibrat 2180 beträgt, ist bei allen Verbindungen der Beispiele 1–7 besser, d.h. höher, und liegt bei etwa 3000 und mehr.

2. Cholesterinsenkung

Der ED 25-Wert, d.h. die für eine Reduktion des Blutcholesteringehaltes um 25% erforderliche Dosis, in mg/kg Körpergewicht, beträgt für Clofibrat 151 und ist bei allen bisher getesteten Verbindungen (1) besser, d.h. geringer.

3. Triglyceridsenkung

Der ED 25-Wert, hier die für eine 25%ige Reduktion des Triglyceridgehaltes im Blut der Versuchstiere erforderliche Dosis in mg/kg Körpergewicht, ist bei allen bisher getesteten Verbindungen (1) besser, d.h. geringer als der entsprechende Aktivitätswert von 222 bei Clofibrat.

Zur besseren Übersicht sind diese Werte in der folgenden Tabelle II zusammengestellt.

Tabelle II

| Verbindung von Beispiel | LD 50 Maus (mg/kg) | Cholesterin ED 25 (mg/kg) | Triglycerid ED 25 (mg/kg) |
|---|---|---|---|
| 1 | 3000 | 80 | <50 |
| 2 | 2960 | 80 | 14 |
| 3 | >3000 | >100 | |
| 4 | >3000 | 41 | 70 |
| 5 | >3000 | 30 | 40 |
| 6 | >3000 | 70 | >100 |
| Vergleich: | | | |
| Clofibrat: | 2180 | 151 | 222 |

Die erfindungsgemässen Verbindungen (1) können im wesentlichen in der für Clofibrat an sich bekannten Weise klinisch verwendet bzw. für erfindungsgemässe pharmazeutische Zubereitungen verwendet werden.

Hierzu können die Verbindungen (1) einzeln oder in Mischung untereinander, gegebenenfalls zusammen mit anderen therapeutisch aktiven Stoffen, in an sich üblicher Weise mit bekannten pharmakologisch zulässigen flüssigen oder festen Trägern und Konfektionierungszusätzen zu flüssigen, halbflüssigen oder festen Zubereitungen, insbesondere für orale Verabreichung, verarbeitet werden, wie dies z.B. in der US-PS 3262850 beschrieben ist. Als Beispiel für Dosierungseinheiten zur medikamentösen Bekämpfung überhöhter Cholesterin- und Triglyceridwerte im Blut ist ein Bereich von etwa 0,05 bis 1 g an aktiver Komponente (1) in erfindungsgemässen pharmazeutischen Zubereitungen zu nennen. Tagesdosen im Bereich von etwa 0,1 bis 10 g an aktiver Komponente (1) pro Patient entsprechen den für die klinische Verwendung von Clofibrat vorgeschlagenen Werten.

## Patentansprüche

1. Alkylenglykolderivate der Formel (1)

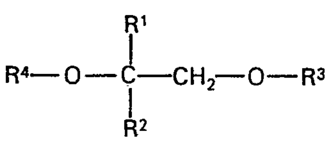

(1)

in der

$R^1$ das Wasserstoffatom, eine Alkylgruppe mit 1 bis 4 C-Atomen oder die Phenyl- oder Benzylgruppe,

$R^2$ das Wasserstoffatom oder eine Alkylgruppe mit 1 bis 4 C-Atomen,

$R^3$ das Wasserstoffatom oder eine Gruppe der Formel

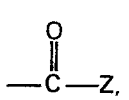

in der Z ein Alkylrest mit 1 bis 10 C-Atomen, die O-Acetyloxyphenyl- oder 3-Pyridinylgruppe ist, und

$R^4$ einen Rest der Formel

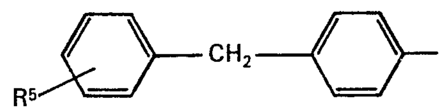

bedeutet, in der $R^5$ das Wasserstoff- oder Chloratom oder die Methylgruppe ist,

mit der Massgabe, dass $R^3$ nicht das Wasserstoffatom ist, wenn $R^4$ die p-Benzylphenylgruppe ist und beide $R^1$, $R^2$ Wasserstoffatome sind.

2. Alkylenglykolderivate nach Patentanspruch 1, dadurch gekennzeichnet, dass $R^5$ das Chloratom in p-Stellung zur Methylenbrücke ist.

3. Alkylenglykolderivate nach Patentanspruch 2, dadurch gekennzeichnet, dass $R^1$ und $R^2$ jeweils das Wasserstoffatom bedeuten.

4. Alkylenglykolderivate nach Patentanspruch 2, dadurch gekennzeichnet, dass $R^1$ und $R^2$ ungleich sind und vorzugsweise unterschiedliche Alkylgruppen, insbesondere Methyl und Aethyl, darstellen.

5. Alkylenglykolderivate nach Patentanspruch 1 in der Form der 2-[4-(4'-Chlorbenzyl)-phenoxy]-alkanole, bei welchen der Alkanolteil 2 bis 10 C-Atome enthält, oder der Ester eines solchen Alkanols mit einer Carbonsäure der Formel Z-C(O)-OH, in der Z die im Anspruch 1 angegebene Bedeutung hat.

6. Verfahren zur Herstellung von Verbindungen der Formel (1) gemäss Patentanspruch 1, gekennzeichnet durch Reduktion einer Verbindung der Formel (2)

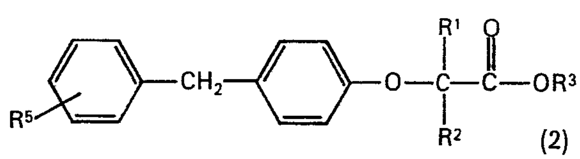

(2)

in der $R^1$ bis $R^5$ die angegebene Bedeutung haben, zum entsprechenden Alkohol der Formel (1), in der $R^3$ das Wasserstoffatom bedeutet, und gegebenenfalls folgende Veresterung des so erhaltenen Alkohols mit einer Carbonsäure der Formel Z-C(O)-OH, in der Z die im Anspruch 1 angegebene Bedeutung hat, oder einem Säurechlorid einer solchen Carbonsäure.

7. Verfahren nach Patentanspruch 6, dadurch gekennzeichnet, dass die Reduktion in einem inerten organischen Lösungsmittel durch Einwirkung von Lithiumaluminiumhydrid erzielt wird.

8. Verfahren zur Herstellung von Verbindungen der Formel (1) gemäss Patentanspruch 1, gekennzeichnet durch Umsetzung eines Phenols der Formel

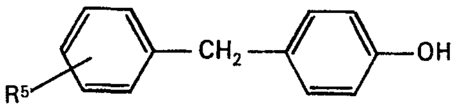

in der $R^5$ die angegebene Bedeutung hat, mit einer Verbindung der Formel

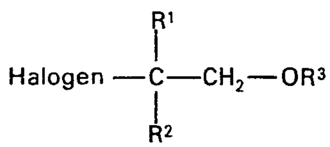

in der $R^1$, $R^2$ und $R^3$ die angegebene Bedeutung haben.

9. Alkylenglykolderivate der Formel (1)

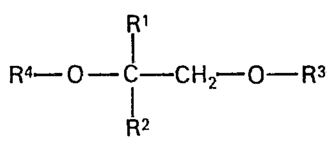

(1)

in der
$R^1$ das Wasserstoffatom, eine Alkylgruppe mit 1 bis 4 C-Atomen oder die Phenyl- oder Benzylgruppe,
$R^2$ das Wasserstoffatom oder eine Alkylgruppe mit 1 bis 4 C-Atomen,
$R^3$ das Wasserstoffatom oder eine Gruppe der Formel

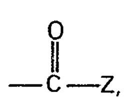

in der Z ein Alkylrest mit 1 bis 10 C-Atomen, die O-Acetyloxyphenyl- oder 3-Pyridinylgruppe ist, und
$R^4$ einen Rest der Formel

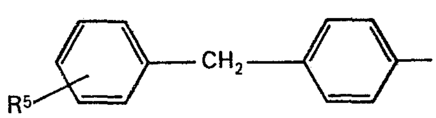

bedeutet, in der $R^5$ das Wasserstoff- oder Chloratom oder die Methylgruppe ist.
Zur Verwendung als lipidsenkender Wirkstoff.

**Claims**

1. Alkylene glycol derivatives of the formula (1)

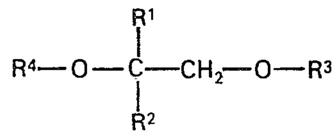

(1)

in which
$R^1$ is the hydrogen atom, an alkyl group having 1-4 C-atoms or the phenyl or benzyl group,
$R^2$ is the hydrogen atom or an alkyl group having 1-4 C-atoms,
$R^3$ is the hydrogen atom or a group of the formula

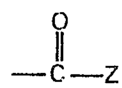

in which Z is an alkyl radical having 1-10 C-atoms, the O-acetyloxyphenyl or 3-pyridinyl group, and
$R^4$ is a radical of the formula

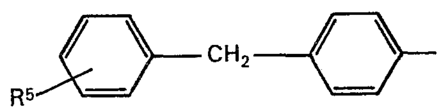

in which $R^5$ is the hydrogen or chlorine atom or the methyl group, with the proviso that $R^3$ is not the hydrogen atom when $R^4$ is the p-benzyl phenyl group and both $R^1$, $R^2$ are hydrogen atoms.

2. Alkylene glycol derivatives according to claim 1, characterized in that $R^5$ is the chlorine atom in p-position to the methylene link.

3. Alkylene glycol derivatives according to claim 2, characterized in that $R^1$ and $R^2$ each signify the hydrogen atom.

4. Alkylene glycol derivatives according to claim 2, characterized in that $R^1$ and $R^2$ are unequal and preferably represent different alkyl groups, notably methyl and ethyl.

5. Alkylene glycol dervatives according to claim 1 in the form of the 2-[4-(4'-chlorobenzyl)-phenoxyl]-alkanols in which the alkanol moiety contains 2-10 C-atoms, or of the esters of such an alkanol with a carboxylic acid of the formula Z-C(O)-OH in which Z has the meaning given in claim 1.

6. A process for preparing compounds of the formula (1) according to claim 1, characterized by the reduction of a compound of the formula (2)

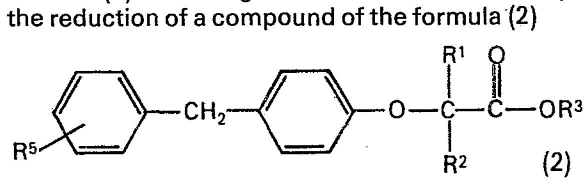

(2)

in which $R^1$ to $R^5$ have the stated significance, to the corresponding alcohol of the formula (1) in which $R^3$ represents the hydrogen atom, and by an optional subsequent esterification of the alcohol thus obtained with a carboxylic acid of the formula Z-C(O)-OH in which Z has the meaning

given in claim 1 or with an acid chloride of such a carboxylic acid.

7. The process of claim 6, characterized in that the reduction is achieved in an inert organic solvent by the action of lithium aluminum hydride.

8. A process for preparing compounds of the formula (1) according to claim 1, characterized by reacting a phenol of the formula

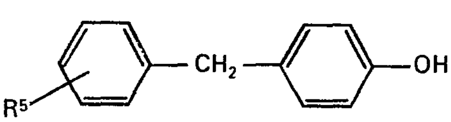

in which $R^5$ has the stated significance, with an alcohol of the formula

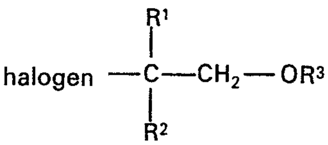

in which $R^1$, $R^2$ and $R^3$ have the stated significance.

9. Alkylene glycol derivatives of the formula (1)

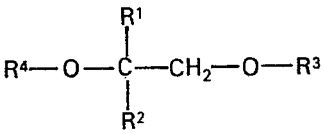

in which
$R^1$ is the hydrogen atom, an alkyl group having 1–4 C-atoms or the phenyl or benzyl group,
$R^2$ is the hydrogen atom or an alkyl group having 1–4 C-atoms.
$R^3$ is the hydrogen atom or a group of the formula

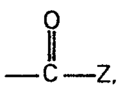

in which Z is an alkyl radical having 1–10 C-atoms, the O-acetyloxyphenyl or 3-pyridinyl group, and
$R^4$ is a radical of the formula

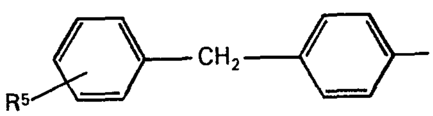

in which $R^5$ is the hydrogen or chlorine atom or the methyl group for use as a lipid lowering agent.

**Revendications**

1. Dérivés d'alkylène-glycols répondant à la formule (1)

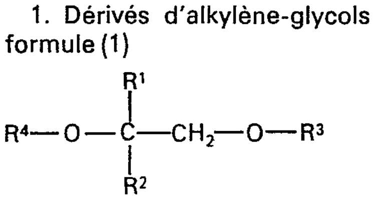
(1)

dans laquelle
$R^1$ représente un atom d'hydrogène, un radical alkyle en $C_1$-$C_4$ ou un radical phényle ou benzyle,
$R^2$ représente un atom d'hydrogène ou un radical alkyle en $C_1$-$C_4$,
$R^3$ représente un atome d'hydrogène ou un radical

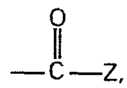

dans lequel Z désigne un radical alkyle en $C_1$-$C_{10}$, un radical O-acétyloxy-phényle ou pyridinyle-3, et
$R^4$ représente un radical répondant à la formule

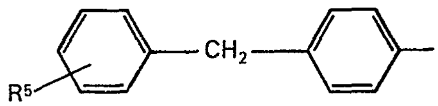

dans laquelle $R^5$ représente un atome d'hydrogène ou de chlore ou un radical méthyle, à condition que $R^3$ ne représente pas un atome d'hydrogène si $R^4$ représente le radical p-benzyle-phényle et $R^1$ et $R^2$ représente chacun un atome d'hydrogène.

2. Dérivés d'alkylène-glycols selon la revendication 1, caractérisés en que $R^5$ représente un atome de chlore en position para relativement au pont méthylène.

3. Dérivés d'alkylène-glycols selon la revendication 2, caractérisés en ce que $R^1$ et $R^2$ représentent chacun un atome d'hydrogène.

4. Dérivés d'alkylène-glycols selon la revendication 2, caractérisés en ce que $R^1$ et $R^2$ sont différents l'un de l'autre et représentent de préférence des radicaux alkyles différents, en particulier méthyle et éthyle.

5. Dérivés d'alkylène-glycols selon la revendication 1 en forme des [(chloro-4' benzyle)-4 phénoxyl]-2 alcanols, dans lesquels la portion alcanolique contient 2–10 atomes C, ou en forme des esters d'un tel alcanol avec un acide carboxylique répondant à la formule Z-C(O)OH, dans laquelle Z réponds à la définition donnée à la revendication 1.

6. Procédé de préparation de composés de formule (1) selon la revendication 1, caractérisé en ce qu'on réduit un composé de formule (2)

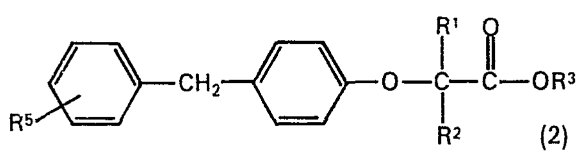
(2)

dans laquelle $R^1$ à $R^5$ ont les significations données à la revendication 1, en l'alcool correspondant de formule (1), dans lequel $R^3$ désigne un atome d'hydrogène, puis on estérifie éventuellement l'alcool ainsi obtenu avec un acide carboxylique répondant à la formule Z-C(O)OH, dans laquelle Z réponds à la définition donnée à la revendication 1, ou avec un chlorure d'un tel acide carboxylique.

7. Procédé selon la revendication 6, caractérisé en ce qu'on effectue la réduction au moyen du

tétrahydruro-aluminate de lithium dans un solvant organique inerte.

8. Procédé de préparation de composés de formule (1) selon la revendication 1, caractérisé en ce qu'on fait réagir un phénol répondant à la formule

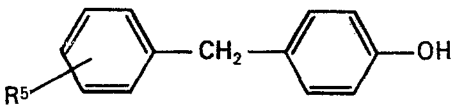

dans laquelle $R^5$ réponds à la définition donnée, avec un alcool répondant à la formule

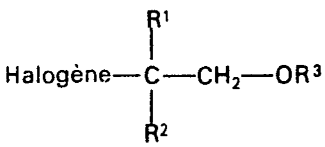

dans laquelle $R^1$, $R^2$ et $R^3$ répondent à la définition donnée.

9. Dérivés d'alkylène-glycols répondant à la formule (1)

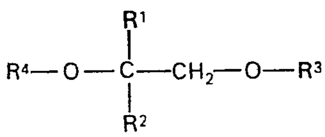

(1)

dans laquelle
$R^1$ représente un atome d'hydrogène, un radical alkyle en $C_1$-$C_4$ ou un radical phényle ou benzyle,
$R^2$ représente un atome d'hydrogène ou un radical alkyle en $C_1$-$C_4$,
$R^3$ représente un atome d'hydrogène ou un radical

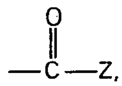

dans lequel Z désigne un radical alkyle en $C_1$-$C_{10}$, un radical O-acétyloxy-phényle ou pyridinyle-3, et
$R^4$ représente un radical répondant à la formule

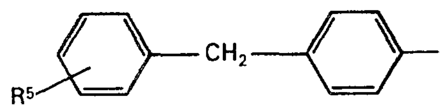

dans laquelle $R^5$ représente un atome d'hydrogène ou de chlore ou un radical méthyle, pour l'utilisation comme agents hypolipémiants.